Europäisches Patentamt

European Patent Office

Office européen des brevets

(19)

(11) Veröffentlichungsnummer: **0 138 755**

**B1**

(12) **EUROPÄISCHE PATENTSCHRIFT**

(45) Veröffentlichungstag der Patentschrift:
04.02.87

(21) Anmeldenummer: 84810395.8

(22) Anmeldetag: 13.08.84

(51) Int. Cl.⁴: **C 07 C 143/60, C 07 C 139/14**

(54) Verfahren zur Isolierung von 2-Naphtylamin-3,6,8-trisulfonsäure als Monokalium- bzw. Monoammoniumsalz.

(30) Priorität: 17.08.83 CH 4486/83

(43) Veröffentlichungstag der Anmeldung:
24.04.85 Patentblatt 85/17

(45) Bekanntmachung des Hinweises auf die Patenterteilung:
04.02.87 Patentblatt 87/6

(84) Benannte Vertragsstaaten:
CH DE FR GB IT LI

(56) Entgegenhaltungen:
EP-A-0 034 398
DE-A-2 757 412
DE-A-3 020 526
DE-A-3 135 391

HOUBEN-WEYL "Methoden der organischen
Chemie", 4. Auflage, Band IX, 1955 GEORG THIEME
VERLAG, Stuttgart, Seiten 478-485

(73) Patentinhaber: CIBA- GEIGY AG, Klybeckstrasse
141, CH- 4002 Basel (CH)

(72) Erfinder: Blattner, Rudolf, Dr., Rheinmattenweg 14,
D-7888 Rheinfelden 4 (DE)

## 0 138 755

### Beschreibung

Die Erfindung betrifft ein Verfahren zur Isolierung von 2-Naphthyl-amin-3,6,8-trisulfonsäure als Monokalium- bzw. Monoammoniumsalz, aus dem bei der Sulfonierung der entsprechenden Naphthylaminmono- bzw. -disulfonsäure in Oleum anfallenden Sulfonierungsgemisch und das nach dem Verfahren isolierte 2-Naphthylamin-3,6,8-trisulfonsäure-Monokalium-bzw. Monoammoniunsalz.

Naphthylamintrisulfonsäuren sind wichtige Zwischenprodukte für die Herstellung von Farbstoffen. Sie werden allgemein durch Sulfonieren der entsprechenden Disulfonsäuren mittels Oleum hergestellt.

Ferner sind die Trisulfonsäuren auch durch Disulfonieren der entsprechenden Naphthylaminmonosulfonsäuren erhältlich, und zwar die 2-Naphthylamin-3,6,8-trisulfonsäure aus der 2-Naphthylamin-6-sulfonsäure und die 1-Naphthylamin-2,5,7-trisulfonsäure aus der 1-Naphthylamin-5-sulfonsäure [Houben-Weyl, Methoden der organischen Chemie 9, 478-485 (1955)].

Zur Aufarbeitung wurde das Sulfonierungsgemisch bislang in Wasser eingetragen und die Naphthylamintrisulfonsäure durch Zusatz von Ammoniumsulfat, Natrium- oder Kaliumsulfat ausgesalzen. Bei dieser Art der Aufarbeitung, bei der grosse Mengen an Sulfat benötigt werden, fällt die Naphthylamintrisulfonsäure vor allem als Diammonium- bzw. Dialkalisalz an. Das Aussalzen führt zu schwer filtrierbaren, stark salzhaltigen Produkten, erschwert die Rückgewinnung der Schwefelsäure und hat zudem eine erhebliche Abwasserbelastung zur Folge.

Gefunden wurde, dass sich die Nachteile der bisherigen Arbeitsweise im Falle der 2-Naphthylamin-3,6,8-trisulfonsäure vermeiden lassen, wenn man dafür sorgt, dass diese zu Beginn der Aufarbeitung in Form des Monokalium- bzw. Monoammoniumsalzes vorliegt, ferner beim Verdünnen des Sulfonierungsgemisches eine definierte Schwefelsäurekonzentration eingestellt, auf das Aussalzen verzichtet und die so erhaltene heisse verdünnte Lösung oder Suspension langsam abgekühlt und während dessen mit einem Dispergiermittel versetzt wird. Bei dieser Art der Aufarbeitung wird die 2-Naphthylamin-3,6,8-trisulfon-säure (als Monokalium-bzw. Monoammoniumsalz) nahezu vollständig in Form eines leichtfiltrierbaren Kristallbreies abgeschieden. Die Abfallsäure ist salzfrei und lässt sich problemlos aufarbeiten.

Gegenstand der Erfindung ist somit ein Verfahren zur Isolierung von 2-Naphthyl-amin-3,6,8-trisulfonsäure als Alkali- oder Ammoniumsalz aus dem bei der Sulfonierung der entsprechenden Naphthylaminmono- oder -disulfonsäure in Oleum anfallenden Sulfonierungsgemisch, dadurch gekennzeichnet, dass man von einem Sulfonierungsgemisch ausgeht, das die Naphthylamintrisulfonsäure als Monokalium- oder Monoammoniumsalz enthält, dieses in Wasser oder verdünnte Schwefelsäure einträgt, so dass die Schwefelsäurekonzentration in der verdünnten wässrigen Lösung bzw. Suspension des Sulfonierungsgemisches 30 bis 77 Gew.% beträgt, dabei die Temperatur auf 90° bis 125°C ansteigen lässt, anschliessend die Lösung bzw. Suspension langsam auf eine Temperatur von unter 40°C abkühlt, während des Abkühlens mindestens 0,1 Gew.%, bezogen auf Naphthylamintrisulfonsäure, eines Dispergiermittels zusetzt und das abgeschiedene Monokalium- bzw. Monoammoniumsalz der 2-Naphthylamin-3,6,8-tri-sulfonsäure isoliert.

Die Aufarbeitung der 2-Naphthylamin-3,6,8-trisulfonsäure in Form des Monokalium- bzw. Monoammoniumsalzes aus 30- bis 77 gew.%iger Schwefelsäure bei Temperaturen unter 40°C erlaubt überraschenderweise die Isolierung eines einheitlichen Produkts auf einfache Art und Weise in hoher Reinheit und guter Ausbeute, da das Monokalium- bzw. Monoammoniumsalz unter den genannten Bedingungen ein Löslichkeitsminimum aufweist.

Die Löslichkeit des Monokaliumsalzes der 2-Naphthylamin-3,6,8-tri-sulfonsäure in Schwefelsäure unterschiedlicher Konzentration, bestimmt bei einer Temperatur von 30°C, ist der folgenden Tabelle zu entnehmen.

| Konzentration der $H_2SO_4$ in Gew.% | 21,2 35,9 41,2 46,0 51,1 60,3 73,2 77,5 80,2 87,5 |
|---|---|
| Mengen an gelöstem K-Salz in Gew.% | 3,5  1,6  0,4  0,2  ~0,1  ~0,1  ~0,1  0,6  4,4  >11 |

Das Monokalium- bzw. Monoammoniumsalz der 2-Naphthylamin-3,6,8-tri-sulfonsäure erhält man entweder so, dass man bereits bei der Sulfonierung vom Monokalium- bzw. Monoammoniumsalz der entsprechenden

2

0138755

Naphthylaminmono- oder -disulfonsäure ausgeht oder aber das am Ende der Sulfonierung erhaltene Reaktionsgemisch mit einer zur Bildung des Monokalium- bzw. Monoammoniumsalzes ausreichenden Menge an Kalium- bzw. Ammoniumsulfat versetzt. Ferner kann man die benötigte Menge Kalium- oder Ammoniumsulfat auch mit dem zur Verdünnung vorgesehenen Wasser bzw. der verdünnten Schwefelsäure vorlegen. Anstelle von Kalium- oder Ammoniumsulfat können auch andere Kalium- oder Ammoniumsalze oder Kalium- bzw. Ammoniumionen abgebenden Mittel, wie beispielsweise wässrige Ammoniaklösung, verwendet werden.

Bevorzugt geht man von einem Sulfonierungsgemisch aus, das durch Umsetzen des Monokaliumsalzes der 2-Naphthylamin-6,8-disulfonsäure in Oleum erhalten wird.

Die Einstellung der Schwefelsäurekonzentration kann in beliebiger Weise vorgenommen werden, beispielsweise durch Einrühren des Reaktionsgemisches in gegebenenfalls schwefelsäurehaltiges Wasser oder umgekehrt. Zur Verdünnung des Sulfonierungsgemisches verwendet man soviel Wasser oder verdünnte Schwefelsäure, dass am Ende die Schwefelsäurekonzentration in der so erhaltenen wässrigen Lösung oder Suspension 30 bis 77 Gew.%, vorzugsweise 40 bis 70 Gew.%, beträgt. - Die Konzentrationsangaben beziehen sich hier auf die flüssige Phase ohne Feststoffanteil.- Dabei geht man zweckmässigerweise so vor, dass man die nötige Wasser- bzw. Schwefelsäuremenge vorlegt und das Sulfonierungsgemisch mit einer solchen Geschwindigkeit zudosiert, dass die Temperatur der entstehenden Lösung bzw. Suspension durch die freiwerdende Verdünnungswärme ohne zusätzliches Erwärmen oder Kühlen auf 90° bis 125°C ansteigt. Selbstverständlich kann man das Sulfonierungsgemisch auch langsamer oder schneller eintragen, es muss dann lediglich durch Heizen oder Kühlen der, Lösung bzw. Suspension eine Temperatur im vorgegebenen Bereich eingestellt werden.

Zur Erzielung einer guten Kristallform sind Temperaturen des verdünnten Sulfonierungsgemisches von 90° bis 125°C vorteilhaft. Nach einer Verweilzeit von ca. einer Stunde wird unter langsamem Rühren auf eine Temperatur von unter 40°C abgekühlt. Die Kühlung erfolgt dabei bevorzugt so, dass die Temperatur mit einer Geschwindigkeit von 0,2 bis 1°C/min sinkt. Die Kühlung kann mit einer konstanten Kühlgeschwindigkeit erfolgen, oder auch in 2 oder mehreren Phasen mit jeweils konstanter aber voneinander verschiedener Geschwindigkeit. Vorteilhaft wählt man zu Anfang eine langsamere Kühlgeschwindigkeit, damit keine übersättigte Lösung entsteht und der gelöste Produktanteil unter Bildung von möglichst wenig Feinkorn auskristallisiert. Die Kühlung erfolgt beispielsweise über den äusseren Mantel des Rührkessels oder mittels eines Tauchkühlers, bei vorteilhaft niedriger Rührerdrehzahl.

Während des Abkühlvorganges versetzt man die schwefelsaure Lösung bzw. Suspension des 2-Naphthylamin-3,6,8-trisulfonsäure-Monokalium-bzw. Ammoniumsalzes mit einem Dispergiermittel. Man verwendet mindestens 0,1 Gew.%, vorzugsweise 0,2 bis 5 Gew.%, bezogen auf Naphthylamintrisulfonsäure, eines anionischen oder kationischen, insbesondere jedoch eines nichtionischen Dispergiermittels. In Frage kommen ferner auch Amphotenside. Je nach Säurestabilität des Dispergiermittels, wird dieses bereits in die noch heisse Lösung bzw. Suspension gegeben oder erst bei tieferer Temperatur zugesetzt. Zweckmässigerweise erfolgt die Zugabe bei einer Temperatur unterhalb von 60°C.

Als nichtionische Dispergiermittel kommen in erster Linie Aethylenoxid-Addukte, vor allem Alkylphenol-, Fettalkohol-, Fettamin- oder Fettsäureäthoxylate oder auch Aethylenoxid/Propylenoxid-Mischpolymerisate in Frage. In den Aethylenoxid-Addukten kann ein Teil der Aethylenoxideinheiten durch Propylenoxideinheiten ersetzt sein.

Als Aethylenoxid-Addukte sind im einzelnen genannt:

a) Umsetzungsprodukte von gesättigten und/oder ungesättigten Fettalkoholen mit 6 bis 20 Kohlenstoffatomen, mit 5 bis 30 Mol Aethylenoxid je Mol Hydroxylgruppe;

b) Umsetzungsprodukte von Alkylphenolen mit 4 bis 12 Kohlenstoffatomen im Alkylrest, mit 5 bis 20 Mol, vorzugsweise 8 bis 15 Mol, Aethylenoxid je Mol phenolische Hydroxylgruppe;

c) Umsetzungsprodukte von gesättigten und/oder ungesättigten Fettaminen mit 14 bis 20 Kohlenstoffatomen mit 5 bis 20 Mol Aethylenoxid je Mol Aminogruppe;

d) Umsetzungsprodukte von gesättigten und/oder ungesättigten Fettsäuren mit 14 bis 20 Kohlenstoffatomen, mit 5 bis 20 Mol Aethylenoxid je Mol Carboxylgruppe.

Von diesen Aethylenoxid-Addukten sind die unter b) genannten Umsetzungsprodukte bevorzugt. Gute Ergebnisse werden ferner mit den unter a) genannten Umsetzungsprodukten erzielt, wobei sich hier insbesondere auch solche Typen bewähren, die durch Anlagern von Aethylenoxid und Propylenoxid an Fettalkohole oder Gemische von Fettalkoholen verschiedener Kettenlänge erhalten werden.

Auch Gemische der Aethylenoxid-Addukte nach a), b), c) und d) untereinander sind verwendbar. Diese Gemische erhält man durch Mischen einzelner Umsetzungsprodukte oder direkt durch Aethoxylierung eines Gemisches der den Addukten zugrundeliegenden Verbindungen.

Als gesättigte und/oder ungesättigte Fettalkohole kommen für a) Dodecanol, Palmitylalkohol, Stearylalkohol, oleylalkohol oder Talgfettalkohole, vorzugsweise Hexanol, 2-Aethylhexanol und Decanol in Betracht.

Als Alkylphenole für b) sind Butylphenol, Hexylphenol, vor allem jedoch Isooctylphenol, p-tert.Octylphenol, Nonylphenol und Dodecylphenol zu nennen.

Als Fettamine für c) kommen z.B. neben Stearylamin, Palmitylamin und vor allem Oleylamin in Betracht.

Für d) sind als gesättigte und/oder ungesättigte Fettsäuren z.B. Palmitinsäure, vor allem Stearinsäure und Oelsäure zu nennen.

Die Aethylenoxid-Addukte sind bekannt oder können nach an sich bekannten Verfahren hergestellt werden

3

(siehe z.B. N. Schönfeldt, Grenzflächenaktive Aethylenoxid-Addukte; Wissenschaftliche Verlagsgesellschaft mbH, Stuttgart, 1976).

Als Aethylenoxid/Propylenoxid-Mischpolymerisate verwendet man beispielsweise Blockpolymere mit mittelständiger Polypropylenglykol-Einheit und einem Molgewicht von 1'000 bis 10'000.

Bei den anionischen Dispergiermitteln handelt es sich um die handelsüblichen Dispergiermittel für wässrige Suspensionen und Dispersionen, z.B. um Kondensationsprodukte von aromatischen Sulfonsäuren mit Formaldehyd, wie z.B. Naphthalinsulfonsäure-Formaldehydkondensate, oder um Ligninsulfonate, z.B. die unter der Bezeichnung Sulfitablauge erhältlichen Verbindungen.

Als kationische Tenside sind beispielsweise quaternäre Alkylammoniumhalogenide mit mindestens einem $C_8$ bis $C_{25}$ Alkylrest und langkettige Alkylpyridiniumhalogenide genannt.

Als Amphotenside werden in vorliegendem Verfahren in erster Linie solche vom Betaintyp eingesetzt, wie z.B. das Betain selbst, das als Anhydrid, Hydrochlorid oder auch Monohydrat verwendet wird.

Durch den Zusatz an Dispergiermittel ist das verdünnte Sulfonierungsgemisch auch noch bei Temperaturen < 30°C gut rührbar und das 2-Naphthylamin-3,6,8-trisulfonsäure-Monokalium- bzw. Monoammoniumsalz wird in einer gut filtrierbaren Form erhalten.

Ist das verdünnte Sulfonierungsgemisch auf eine Temperatur < 40°C heruntergekühlt, im allgemeinen auf eine Temperatur von 10° bis 30°C, da ein Abkühlen auf Temperaturen unter 10°C unwirtschaftlich ist, wird die in Form des Monokalium- bzw. Monoammoniumsalzes praktisch vollständig ausgefallene 2-Naphthylamin-3,6,8-trisulfonsäure von der Mutterlauge abgetrennt. Dies geschieht zweckmässigerweise durch Filtration. Der so erhaltene schwefelsäurehaltige Filterkuchen kann durch Waschen mit 30- bis 70%iger Schwefelsäure gereinigt und der Presskuchen ausgeblasen werden. Da das 2-Naphthylamin-3,6,8-tri-sulfonsäure-Kalium- bzw. Ammoniumsalz in hoher Reinheit anfällt, ist auch dessen direkte Weiterverarbeitung beispielsweise zu Farbstoffen möglich, wobei man ohne irgendwelche weitere Reinigungsoperationen zum Entfernen unerwünschter Nebenprodukte auskommt.

Das erfindungsgemässe Verfahren kann z.B. wie folgt durchgeführt werden. In einem Rührkessel wird die zum Verdünnen des Sulfonierungsgemisches auf einen Schwefelsäuregehalt von 40 bis 70 Gew.% benötigte Wassermenge vorgelegt. Anschliessend trägt man das auf bekannte Art und Weise durch Sulfonieren des Monokaliumsalzes der 2-Naphthylamin-6,8-disulfonsäure erhaltene Sulfonierungsgemisch ein, wobei die Temperatur auf ca. 100° bis 115°C ansteigt. Die heisse Suspension, in der ein Teil des Produktes in gelöster Form vorliegt, kühlt man anschliessend mit einer Geschwindigkeit von ca. 0,5°C/min ab und setzt bei einer Temperatur von etwa 50°C ein nichtionisches Dispergiermittel zu (ca. 1 Gew.%, bezogen auf Naphthylamintrisulfonsäure). Ist eine Temperatur von ca. 25°C erreicht, filtriert man das in kristalliner Form vorliegende Naphthyamintrisulfonsäure-Monokaliumsalz ab. Ein Nachwaschen des Filterkuchens ist nicht erforderlich. Das auf diese Weise isolierte Produkt zeichnet sich durch eine hohe Reinheit aus; der Gehalt an Nebenprodukten liegt unter 0,5 Gew.%.

Die Qualität der nach dem vorliegenden Verfahren in Form des Monokalium- bzw. Monoammoniumsalzes isolierten 2-Naphthylamin-3,6,8-tri-sulfonsäure ist deutlich besser, als bei Isolierung der freien Trisulfonsäure oder auch des Dikalium- bzw. Diammoniumsalzes bzw. der gemischten Kalium/Ammoniumsalze durch Aussalzen. Zudem erzielt man eine Ausbeutesteigerung von ca. 5%. Die Erfindung wird durch das folgende Beispiel erläutert; Prozente bedeuten Gewichtsprozente.


**Beispiel**

a) Sulfonierung: In 6.020 g ca. 40%iges Oleum, hergestellt aus 3.580 g 25%igem und 2.440 g 66%igem Oleum, werden unter Stickstoffspülung 1.688,5 g 2-Naphthylamin-6,8-disulfonsäure-Monokaliumsalz so eingetragen, dass die Temperatur nur langsam ansteigt. Nachdem die gesamte Menge an Disulfonsäure-Monokaliumsalz eingetragen ist, erhitzt man das Reaktionsgemisch auf eine Temperatur von ca. 140°C. Nach einer Reaktionszeit von 5 bis 6 Stunden lässt man das Sulfonierungsgemisch abkühlen und beginnt mit der Aufarbeitung.

b) Isolierung: Zur Aufarbeitung lässt man das Sulfonierungsgemisch langsam in 9.000 g Wasser einlaufen, das zuvor auf 30°C erwärmt wurde. Dabei steigt die Temperatur auf 105°C und wird anschliessend durch Kühlen in einem Bereich von 105° bis 109°C gehalten. Die zum Nachspülen des Reaktionskessels, in dem die Sulfonierung durchgeführt wurde, verwendete 98%ige Schwefelsäure (200 g), lässt man ebenfalls in die vorgelegte Wassermenge einlaufen. Der Schwefelsäuregehalt des verdünnten Sulfonierungsgemisches beträgt 43-44%. Die so erhaltene gelbbraune Suspension kühlt man anschliessend unter langsamem Rühren mit einer Geschwindigkeit von 0,5°C/min auf eine Temperatur von 28°C ab. Bei ca. 50°C versetzt man das Sulfonierungsgemisch mit 15 g p-tert.Octylphenoläthoxylat (p-tert.Octylphenol + 8,2 Aethylenoxid). Nach Erreichen der Filtrationstemperatur wird das ausgefallene Monokaliumsalz der 2-Naphthylamin-3,6,8-trisulfonsäure abfiltriert und der Filterkuchen trockengesaugt.

Man erhält 2.740,5 g schwefelsaures Produkt mit einem Gehalt von 70 % (Ausbeute: 92% d.Th.); Gehalt an Nebenprodukten: < 0,5%.

Verwendet man anstelle des p-tert.Octylphenoläthoxylats, bei sonst gleicher Arbeitsweise, eines der folgenden Dispergiermittel und zwar in einer Konzentration von 0,2% bezogen auf die Reaktionsmasse, so

**0 138 755**

erhält man ebenfalls einen nicht oder nur leicht thixotropen Kristallbrei, der sich gut filtrieren lässt. Es ist dies zum einen ein weiteres nichtionisches Tensid, ein Fettalkohol($C_6$-$C_{10}$)-äthoxy-lat/propoxylat und zum anderen ein kationisches Tensid, das N,N-Dimethyl-N-benzyl-alkyl($C_8$-$C_{18}$)ammoniumchlorid.

**Patentansprüche**

1. Verfahren zur Isolierung von 2-Naphthylamin-3,6,8-trisulfonsäure als Alkali- oder Ammoniumsalz, aus dem bei der Sulfonierung der entsprechenden Naphthylaminmono- oder -disulfonsäure in Oleum anfallenden Sulfonierungsgemisch, dadurch gekennzeichnet, dass man von einem Sulfonierungsgemisch ausgeht, das die Naphthylamintrisulfonsäure als Monokalium- bzw. Monoammoniumsalz enthält, dieses in Wasser oder verdünnte Schwefelsäure einträgt, so dass die Schwefelsäurekonzentration in der verdünnten wässrigen Lösung bzw. Suspension des Sulfonierungsgemisches 30 bis 77 Gew.% beträgt, dabei die Temperatur auf 90° bis 125°C ansteigen lässt, anschliessend die Lösung bzw. Suspension langsam auf eine Temperatur von unter 40°C abkühlt, während des Abkühlens mindestens 0,1 Gew.%, bezogen auf Naphthylamintrisulfonsäure, Dispergiermittel zusetzt und das abgeschiedene Monokalium- bzw Monoammoniumsalz der 2-Naphthylamin-3,6,8-trisulfonsäure isoliert.

2. Verfahren gemäss Anspruch 1, dadurch gekennzeichnet, dass die Schwefelsäurekonzentration in der verdünnten wässrigen Lösung bzw. Suspension des Sulfonierungsgemisches 40 bis 70 Gew.% beträgt.

3. Verfahren gemäss Anspruch 1, dadurch gekennzeichnet, dass man die heisse verdünnte Lösung bzw. Suspension des Sulfonierungsgemisches mit einer Geschwindigkeit von 0,2 bis 1°C/min abkühlt.

4. Verfahren gemäss Anspruch 1, dadurch gekennzeichnet, dass man das Dispergiermittel zugibt, wenn die Lösung bzw. Suspension eine Temperatur unter 60°C aufweist.

5. Verfahren gemäss Anspruch 1, dadurch gekennzeichnet, dass man das Dispergiermittel in einer Menge von 0,2 bis 5 Gew.%, bezogen auf Naphthylamintrisulfonsäure, zugibt.

6. Verfahren gemäss Anspruch 1, dadurch gekennzeichnet, dass man ein nichtionisches Dispergiermittel verwendet.

7. Verfahren gemäss Anspruch 6, dadurch gekennzeichnet, dass man als Dispergiermittel ein Aethylenoxidaddukt oder ein Aethylenoxid/Propylenoxidaddukt eines Alkylphenols oder Fettalkohols bzw. Fettalkoholgemisches verwendet.

8. Verfahren gemäss Anspruch 1, dadurch gekennzeichnet, dass man von einem Sulfonierungsgemisch ausgeht, erhalten durch Umsetzen des Monokaliumsalzes der 2-Naphthylamin-6,8-disulfonsäure in Oleum.

**Claims**

1. A process for isolating 2-naphthylamine-3,6,8-trisulfonic acid in the form of the alkali or ammonium salt from the sulfonation mixture resulting from the sulfonation of the corresponding naphthylaminemonosulfonic or -disulfonic acid in oleum, which process comprises starting from a sulfonation mixture which contains the naphthylaminetrisulfonic acid in the form of the monopotassium or monoammonium salt, charging said mixture into water or dilute sulfuric acid such that the concentration of the sulfuric acid in the dilute aqueous solution or suspension of the sulfomation mixture is 30 to 77% by weight, simultaneously allowing the temperature to rise to 90° to 125°C, subsequently slowly cooling said solution or suspension to a temperature below 40°C, during said cooling adding at least 0.1% by weight, based on naphthylaminetrisulfonic acid, of a dispersant, and isolating the precipitated monopotassium or monoammonium salt of 2-naphthylamine-3,6,8-trisulfonic acid.

2. A process according to claim 1, wherein the concentration of sulfuric acid in the dilute aqueous solution or suspension of the sulfonation mixture is 40 to 70% by weight.

3. A process according to claim 1, wherein the hot dilute solution or suspension of the sulfonation mixture is cooled at a rate of 0.2 to 1°C/min.

4. A process according to claim 1, wherein the dispersant is added when the temperature of the solution or suspension is below 60°C.

5. A process according to claim 1, wherein the dispersant is added in an amount of 0.2 to 5% by weight, based on naphthylaminetrisulfonic acid.

6. A process according to claim 1, wherein a nonionic dispersant is used.

7. A process according to claim 6, wherein the dispersant is an ethylene oxide adduct or an adduct of ethylene oxide/propylene oxide with an alkylphenol or fatty alcohol or mixture of fatty alcohols.

8. A process according to claim 1, wherein a start is made from a sulfonation mixture obtained by reacting the monopotassium salt of 2-naphthylamine-6,8-disulfonic acid in oleum.

5

# 0 138 755

## Revendications

1. Procédé d'isolement de l'acide 2-naphtylamine-3,6,8-trisulfonique sous forme de sel d'ammonium ou de métal alcalin, à partir du mélange de sulfonation qui se forme lors de la sulfonation dans un oléum d'un acide naphtylamine-monosulfonique ou naphtylamine-disulfonique correspondant, caractérisé en ce que l'on part d'un mélange de sulfonation qui contient l'acide naphtylamine-trisulfonique sous forme de sel monopotassique ou monoammonique, on introduit ce mélange dans de l'eau ou de l'acide sulfurique dilué, de façon que la concentration de l'acide sulfurique dans la solution ou suspension aqueuse diluée du mélange de sulfonation soit comprise entre 30 et 77 % en poids, tout en laissant la température augmenter jusqu'à une valeur comprise entre 90 et 125°C, on refroidit ensuite lentement la solution ou la suspension à une température inférieure à 40°C, on ajoute au cours du refroidissement au moins 0,1 % en poids, par rapport à l'acide naphtylaminetrisulfonique, d'un agent dispersant, et on isole le sel monoammonique ou monopotassique précipité de l'acide 2-naphtylamine-3,6,8-trisulfonique.

2. Procédé conforme à la revendication 1, caractérisé en ce que la concentration d'acide sulfurique dans la solution ou suspension aqueuse diluée du mélange de sulfonation est comprise entre 40 et 70 % en poids.

3. Procédé conforme à la revendication 1, caractérisé en ce que l'on refroidit la solution ou suspension diluée très chaude du mélange de sulfonation à une vitesse comprise entre 0,2 et 1°C/minute.

4. Procédé conforme à la revendication 1, caractérisé en ce que l'on ajoute l'agent dispersant lorsque la solution ou la suspension présente une température inférieure à 60°C.

5. Procédé conforme à la revendication 1, caractérisé en ce que l'on ajoute l'agent dispersant en une quantité comprise entre 0,2 et 5 % en poids, par rapport à l'acide naphtylamine-trisulfonique.

6. Procédé conforme à la revendication 1, caractérisé en ce que l'on utilise un agent dispersant non ionique.

7. Procédé conforme à la revendication 6, caractérisé en ce que l'on utilise comme agent dispersant un produit d'addition de l'oxyde d'éthylène, ou un produit d'addition de l'oxyde d'éthylène et de l'oxyde de propylène, sur un alkylphénol, un alcool gras ou un mélange d'alcools gras.

8. Procédé conforme à la revendication 1, caractérisé en ce que l'on part d'un mélange de sulfonation obtenu par réaction du sel monopotassique de l'acide 2-naphtylamine-6,8-disulfonique avec un oléum.